(19) Europäisches Patentamt
European Patent Office
Office européen des brevets

(11) EP 1 833 308 B1

# (12) EUROPÄISCHE PATENTSCHRIFT

(45) Veröffentlichungstag und Bekanntmachung des Hinweises auf die Patenterteilung:
**01.07.2009 Patentblatt 2009/27**

(21) Anmeldenummer: **05818009.2**

(22) Anmeldetag: **29.11.2005**

(51) Int Cl.:
***A23K 1/16*** *(2006.01)* ***A23L 1/30*** *(2006.01)*
***A61K 31/19*** *(2006.01)*

(86) Internationale Anmeldenummer:
**PCT/EP2005/012730**

(87) Internationale Veröffentlichungsnummer:
**WO 2006/058695 (08.06.2006 Gazette 2006/23)**

## (54) STEIGERUNG DES PROTEINANTEILS TIERISCHER ORGANISMEN

INCREASING THE PROTEIN CONCENTRATION OF ANIMAL ORGANISMS

AUGMENTATION DE LA FRACTION PROTEIQUE DES ORGANISMES ANIMAUX

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **01.12.2004 DE 102004058079**

(43) Veröffentlichungstag der Anmeldung:
**19.09.2007 Patentblatt 2007/38**

(73) Patentinhaber: **BASF SE**
**67056 Ludwigshafen (DE)**

(72) Erfinder:
- **KARL, Jörn**
  **67063 Ludwigshafen (DE)**
- **DIEBOLD, Gerd**
  **72768 Reutlingen (DE)**
- **RÖSCH, Markus**
  **55276 Dienheim (DE)**
- **OBERFRANK, Uwe**
  **67354 Römerberg (DE)**

(56) Entgegenhaltungen:
**GB-A- 1 409 945** **US-A- 3 397 990**
**US-A- 5 656 656**

- **DATABASE WPI Section Ch, Week 198637 Derwent Publications Ltd., London, GB; Class B05, AN 1986-242356 XP002373018 & JP 61 171418 A (WAKUNAGA SEIYAKU KK) 2. August 1986 (1986-08-02)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 833 308 B1

## Beschreibung

Die vorliegenden Erfindung betrifft die Verwendung von Tartronsäure oder deren Derivaten zur Herstellung von Zubereitungen die zur Steigerung des Proteinanteils tierischer Organismen geeignet sind. Ein weiterer Gegenstand der Erfindung ist die Verwendung der Tartronsäure oder deren Derivate enthaltende Zubereitungen in Tierfutter oder menschlichen Nahrungsmitteln.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die ersten Hinweise auf eine physiologische Wirkung von Tartronsäure (2-hydroxymalonsäure) auf den tierischen Stoffwechsel wurden bereits in den fünfziger Jahren des letzten Jahrhunderts im Zusammenhang mit Untersuchungen der *de novo* Lipogenese in Ratten gefunden. (Wesson LG., Endocrinology. 1950 Oct;47(4):302-4.) Aus den Ergebnissen dieser Studie wurde gefolgert, dass Tartronsäure die Umwandlung von Kohlenhydraten zu Fettsäuren inhibiert, sofern die Versuchtiere eine fettarme aber kohlenhydratreiche Diät erhalten haben.

Dass eine Übertragbarkeit der von Wesson bei Ratten beobachteten physiologischen Effekte auf Menschen fraglich ist, da aufgrund der hohen Glykogenspeicherkapazität die *de novo* Lipogenese beim Menschen praktisch keine Rolle spielt, wurde durch Untersuchungen in den achtziger Jahren des letzten Jahrhunderts belegt. (Archeson et al., Am J Clin Nutr. 1988 Aug;48(2):240-7).

Neuere Studien belegten allerdings, dass Tartronsäure als L-Malat Analog fungieren kann und somit Malat umsetzende Enzyme inhibiert. So konnte eine direkte inhibitorische Wirkung der Tartronsäure auf das menschliche cytosolische und mitochondriale Malat Enzym (Chang et al.; Arch Biochem Biophys. 1992 Aug 1;296(2):468-73), sowie auf das Malat Enzym, die Malat Dehydrogenase und die ATP:Citrat Lyase aus *Mucor circineiloides* nachgewiesen werden (Savitha, World Journal of Microbiology & Biotechnology 16: 513-518, 2000).

Da bekannt ist, dass die ATP:Citrat Lyase ein für die Fettsäurebiosynthese in Mikroorganismen wichtiges Enzym darstellt, und auch vom Malat Enzym angenommen wird, dass es an der Bereitstellung von Reduktionsäquivalenten für die Fettsäurebiosynthese beteiligt ist, wurde der Effekt von Tartronsäure auf die Fettsäurebiosynthese in dem Pilz *Mucor circineiloides* durch *in vivo* Inhibierungsstudien untersucht. Diese Studien zeigten allerdings, dass die *in vivo* inhibitorische Wirkung der Tartronsäure nicht ausreicht um einen nachweisbaren Effekt auf den Lipidstoffwechsel in diesem Organismus zu haben.

Dass allerdings mit Acylresten veresterte Tartronsäurederivate einen physiologischen Effekt auf den menschlichen Stoffwechsel haben, wird in der Anmeldung DE 2806804 offenbart. Darin ist beschrieben, dass Tartronsäureacylester, da diese als Substratanaloga fungieren, die Aktivität von menschlichen Lipasen inhibieren. Es wird in diesem Dokument spekuliert, dass die aufgenommenen Nahrungsfette infolge der Inhibierung der Lipasen durch die Tartronsäureester nicht mehr gespalten werden können und somit unverdaut ausgeschieden werden.

Ferner ist dem Stand der Technik zu entnehmen, dass Tartronsäure enthaltende Zubereitungen beim Menschen zur Behandlung von Verhomungsstörungen der Haut (US 3,988,470) und Knochenstoffwechselerkrankungen (Caselli et al., J Bone Mineral Res. 1997 Jun;12(6):972-81; WO 94/10127) angewendet werden können.

WO 00/48596 beschreibt Lösungen oder feste Mischungen des Antibiotikums Amoxycillin zur Behandlung von Tieren. In diesen Zubereitungen kann z.B. auch Tartronsäure zur Verbesserung des Geschmacks und der Verdaulichkeit zugefügt werden.

In der Bewerbung eines unter dem Namen "Slim Beauty Capsule" vertriebenen Produktes, welches eine Mischung aus Extrakten verschiedenster Pflanzenarten enthält, wird auf das Vorhandensein von Tartronsäure in diesem Produkt hingewiesen. Ferner wird bemerkt, dass Tartronsäure zusammen mit anderen ungesättigten Fettsäuren einen in der Reduktion der Fettakkumulation resultierenden Effekt auf den menschlichen Stoffwechsel hat.

Gemäß dieser Werbung hat die zusätzlich in den "Slim Beauty Capsules" enthaltende Komponente Chromax eine Muskelmasse steigernde Wirkung.

Trotz eines steigenden Ernährungsbewusstsein ist ein stetiger Anstieg der Zahl der ernährungsbedingten Erkrankungen in unserer Gesellschaft zu verzeichnen.

Die häufigste Folge von falscher Ernährung ist das Übergewicht ("Adipositas"), zumeist eine Folge von verstärkter Aufnahme und folgender Einlagerung von Fett.

Ernährungsbedingte Erkrankungen spielen auch bei Jugendlichen eine immer größere Rolle. Die Kosten für ernährungsbedingte Erkrankungen beliefen sich im Jahr 2001 auf circa 145 Milliarden Mark.

Zwei prinzipielle Strategien können zur Bewältigung dieser Problematik beitragen. Dies ist zum einen die Anwendung vorbeugender Maßnahmen zur Vermeidung der Entstehung ernährungsbedingter Erkrankungen und zum anderen die Behandlung von bereits bestehenden Erkrankungen durch speziellen Diäten auch in Kombination mit geeigneten Hilfspräparaten.

Die zunehmende Sensibilisierung des Verbrauchers in Bezug auf Lebensmittel hat Änderungen in dessen

Kaufverhalten zur Folge.

Die geänderte Nachfrage nach fettarmen Fleisch hat die Zuchtziele in der Nutztierhaltung verändert, so wurde von einem hohen Fett- auf einen hohen Magerfleischanteil umgestellt. Daher ist z.B. die Schlachtkörperqualität, der Muskelfleischanteil und somit der Magerfleischanteil bei Schlachttieren im Hinblick auf die Vermarktung von großer Bedeutung.

Des weiteren gibt es eine große Nachfrage nach Präparaten zur Gewichtsreduktion oder der Vorbeugung der Gewichtssteigerung durch unerwünschte Fetteinlagerungen. In diesem Zusammenhang ist auch die Nachfrage nach "functional food" Produkten gestiegen. Unter "functional food" werden Lebensmittel subsumiert, die durch Zugabe bestimmter Nährstoffe/Zutaten so modifiziert wurden, dass sie spezifische gesundheitliche Nutzen bzw. Vorteile erbringen, z.B. den Erhalt eines angemessenen Körpergewichts unterstützen.

"functional food" sind daher Esswaren, die sich nicht nur besonders günstig auf die bereits bestehenden Gesundheitsrisiken auswirken, sondern außerdem aktiv in den Organismus eingreifen und somit eine Arzneimittel ähnliche Wirkung haben.

Aufgabe der vorliegenden Erfindung war die Identifikation und Bereitstellung einer den Proteingehalt tierischer Organismen beeinflussenden Substanz sowie die Bereitstellung von auf dieser Substanz basierenden Zubereitungen für die Anwendung in der Tier und Humanemährung.

Von besonderem Interesse war es hierbei ein System bereitzustellen, welches es ermöglicht, den Proteinanteil tierischer Organismen zu steigern und somit eine Erhöhung des Magerfleischanteils von Nutztieren. Auch sollte eine Verbesserung der "lean body mass" beim Menschen bewirkt werden, wobei die lean body mass als die Summe aller Gewichtsbestandteile eines tierischen Organismus abzüglich seines Fettanteils zu verstehen ist.

Überraschenderweise wurde gefunden, dass die erfindungsgemäße Aufgabe durch die anspruchsgemäße Verwendung gelöst werden konnte.

Gegenstand der vorliegenden Erfindung ist demnach die Verwendung von Tartronsäure, deren Derivate oder deren Mischungen (im folgenden auch als Verbindungen T" zusammengefasst) zur Herstellung einer Zubereitung geeignet zur Steigerung des Proteinanteils eines tierischen Organismus.

Eine erfindungsgemäß besonders bevorzugte Verbindung T ist die Tartronsäure.

Die erfindungsgemäß verwendeten Zubreitungen können auch Salze der Tartronsäure bzw. Salze der Tartronsäurederivate enthalten. Bevorzugte Salze der Tartronsäure bzw. der Tartronsäurederivate sind Ammonium-, Kalium-, Natrium-, Kalzium- oder Magnesium Salze.

Die Zubereitungen können dabei sowohl lediglich eine Verbindung T als auch Mischungen verschiedener, beispielsweise zwei oder mehr verschiedene Verbindungen T enthalten.

Tartronsäurederivate im Sinne der vorliegenden Erfindung umfassen alle Substanzen der allgemeinen Formel 1:

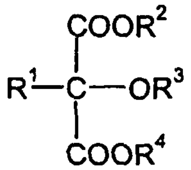

Formel 1

in der die Substituenten und Variablen unabhängig voneinander folgende Bedeutung haben:

$R^1$ Wasserstoff, $C_1$-$C_{20}$-Alkyl-Gruppe;

$R^2$ Wasserstoff, $C_1$-$C_{20}$Acyl-, $C_1$-$C_4$ Alkyl-, $C_1$-$C_4$-Alkoxyethyl-, Allyl- oder p-Methoxybenzyl-Gruppe, ein Alkali oder Erdalkalimetall, ein Ammonium oder $C_1$-$C_{10}$ Alkylammoniumkation;

$R^3$ Wasserstoff, $C_1$-$C_{20}$ Acyl-, $C_1$-$C_4$ Alkyl-, $C_1$-$C_4$-Alkoxyethyl-, Allyl- oder p-Methoxybenzyl-Gruppe, ein Alkali oder Erdalkalimetall, ein Ammonium oder $C_1$-$C_{10}$ Alkylammoniumkation;

$R^4$ Wasserstoff, $C_1$-$C_{20}$Acyl-, $C_1$-$C_4$ Alkyl-, $C_1$-$C_4$-Alkoxyethyl-, Allyl- oder p-Methoxybenzyl-Gruppe, ein Alkali oder Erdalkalimetall, ein Ammonium oder $C_1$-$C_{10}$ Alkylammoniumkation;

Die Verfahren zur Synthese von Verbindungen T sind dem Fachmann bekannt und im Stand der Technik hinreichend beschrieben (siehe z.B. B. Bak, Justus Liebigs Annalen der Chemie, 537, 286 (1939); (P.Chang und S.L. Wu, Chem. Abstr. 52, 14567 (1958); WO 94/10127; Tetrahedron letters, Vol: 39 (18) 1998).

Die Synthese von Verbindungen T kann aber prinzipiell auch unter Anwendung von in der Fachwelt allgemein bekannten Methoden erfolgen.

In einer Ausführungsform können die Zubereitungen zusätzlich zu den Verbindungen T weitere Bestandteile enthalten. Die Wahl der weiteren Bestandteile richtet sich dabei nach dem gewählten Einsatzgebiet der Zubereitungen. Als weitere Bestandteile im Sinne der vorliegenden Erfindung kommen beispielsweise folgende Stoffe in Betracht: weitere organische Säuren, Carotinoide, Spurenelemente, Antioxidantien, Vitamine, Enzyme, Aminosäuren, Mineralstoffe, Emulgatoren, Stabilisatoren, Konservierungsmittel, Antibackmittel und/oder Geschmacksverstärker.

Beispiele für in Betracht kommende Vertreter der genannten Substanzklassen können den jeweils geltenden Listen für Lebensmittelzusatzstoffe nach Europäischer Vorschrift entnommen werden, z.B. der derzeit geltenden EG Richtlinie 95/ 2/ EG.

Im folgenden sind zur Herstellung von erfindungsgemäßen Zubereitungen geeignete weitere Bestandteile aufgeführt:

Diese Bestandteile werden den Zubereitungen, gemäß ihrer unterschiedlichen Eigenschaften und in Abhängigkeit vom gewählten Einsatzgebiet, in unterschiedlichen Mengen beigefügt. Die in Abhängigkeit vom gewählten Einsatzgebiet sinnvollen Kombinationen der Substanzklassen sowie die quantitativen Mischungsverhältnisse sind dem Fachmann bekannt.

Bevorzugt verwendete organischen Säuren sind Ameisensäure, Propionsäure, Milchsäure, Essigsäure und Zitronensäure, besonders bevorzugt sind Ameisensäure, Propionsäure oder Milchsäure.

Im Sinne der vorliegenden Erfindung werden als Carotinoide Tetraterpene verstanden bei denen ein oder zwei Ionon-Ringe durch eine Kohlenstoffkette mit 9 Doppelbindungen verbunden sind und sowohl pflanzlichen als auch tierischen Ursprungs sein können. Als Carotinoide werden auch die sauerstoffhaltigen Xanthophylle verstanden. Beispielhaft seien genannt: Alpha-,Beta-, Gamma-Carotine, Ixin, Norbixin, Capsanthin, Capsorubin, Lycobin, Beta-Apo-8-Carotinal, Carotinsaeure-Aethylester als auch die Xanthophylle Flavoxanthin, Lutein, Kryptoaxanthin, Rubixanthin, Violaxanthin, Rhodoxanthin als auch Canthaxanthin.

Die erfindungsgemäßen Zubereitungen können beispielsweise die folgenden Spurenelemente enthalten: Chrom, Eisen, Fluor, Jod, Kobalt, Kupfer, Mangan, Molybdän, Nickel, Selen, Vanadium, Zink oder Zinn.

Bei den im folgenden aufgeführten E-Nummern handelt es sich um die im der Richtlinie 95/2/EWG gebräuchliche Bezeichnung für Lebensmittelzusatzstoffe.

Als Antioxidantien können z.B. eingesetzt werden, Ascorbinsäure (Vitamin C, E 300), Natrium-L-Ascorbat (E 301), Calcium-L-Ascorbat (E 302), Ascorbylpalmitat (E 304), Butylhydroxyanisol (E 320), Butylhydroxytoluol (E 321), Calcium-Dinatrium-EDTA (E 385), Gallate wie z.B. Propylgallat (E 310), Octylgallat (E 311), Dodecylgallat (Laurylgallat) (E 312), Isoascorbinsäure (E 315), Natriumisoascorbat (E 316), Lecithin (E 322), Milchsäure (E 270), Mehrfach-Phosphate z.B. Diphosphate (E 450), Triphosphate (E 451), Polyphosphate (E 452), Schwefeldioxid (E 220), Natriumsulfit (E 221), Natriumbisulfit (E 222), Natriumdisulfit (E 223), Kaliumsulfit (E 224), Calciumsulfit (E 226), Calciumhydrogensulfit (E 227), Kaliumbisulfit (E 228), Selen, Tocopherole (Vitamin E, E 306) wie z.B. Alpha-Tocopherol (E 307), Gamma-Tocopherol (E 308), Delta-Tocopherol (E 309) und alle Tocotrienole, Zinn-II-Chlorid (E 512), Zitronensäure (E 330), Natriumcitrat (E 331), Carotinoide, Vitamin A sowie Kaliumcitrat (E 332).

Als Vitamine kommen sowohl fettlösliche als auch wasserlösliche Vitamine in Betracht. Bei den fettlöslichen Vitaminen sind beispielsweise zu nennen: Vitamin A (Retinol), Vitamin D (Calciferole), Vitamin E (Tocopherole und Tocotrienole), Vitamin K (Phyllochinone und Menachinone), wobei die Vitamine A und E bevorzugt sind.

Bei den wasserlöslichen Vitaminen sind beispielsweise zu nennen: Vitamin B1 (Thiamin),Vitamin B2 (Riboflavin), Vitamin B5 (Pantothensäure), Vitamin B6 (Pyridoxin), Vitamin B12 (Cobalamin), Vitamin C (Ascorbinsäure), Vitamin H (Biotin), Folsäure und Niacin, wobei die Vitamine B2 und C bevorzugt sind.

Die Zubereitungen können auch Enzyme enthalten. Beispielhaft seien genannt: Amylasen, Proteasen und Invertasen,

Im Sinne dieser Erfindung in Betracht kommende Aminosäuren sind z.B. Glutaminsäure, L-Carnitin, L-Glutamin, L-Taurin, L- Asparaginsäure, L-Glycin, L-Lysin, DL-Phenylalanin, L-Tryptophan, Tyrosin, L-Arginin, L-Cystein, L-Leucin, L- Methionin, L-Alanin, L-Serin, L-Threonin, L-Citrullin, L-Valin, L- Histidin, L-Isoleucin, L-Omithin oder L-Prolin.

Besonders bevorzugt sind die essentiellen Aminosäuren wie z.B. L-Isoleucin, L-Leucin, L-Lysin, L- Methionin, DL-Phenylalanin, L-Threonin, L-Tryptophan und L-Valin, ganz besonders bevorzugt sind die in der Tierernährung wichtigen Aminosäuren L-Lysin, DL- Methionin oder L-Threonin.

Mineralstoffe im Sinne dieser Erfindung sind z.B. Natrium, Kalium, Magnesium, Calcium, Phosphor, Eisen und Zink.

Als Emulgatoren können beispielsweise folgende Substanzen verwendet werden: E 420 Sorbit, E 420ii Sorbitsirup, E 421 Mannit, E 422 Glycerin, E 431 Polyoxyethylen(40)stearat, E 432 Polyoxyethylen-sorbitan-monolaurat / Polysorbat 20, E 433 Polyoxyethylen-sorbitan-monooleat / Polysorbat 80, E 434 Polyoxyethylen-sorbitanmonopalmitat / Polysorbat 40, E 435 Polyoxyethylen-sorbitan-monostearat / Polysorbat 60, E 436 Polyoxyethylen-sorbitan-tristearat / Polysorbat 65, E 440 Pektine, E 440i Pektin, E 440ii Amidiertes Pektin, E 442 Ammoniumphoshatide, E 444 Saccharoseacetat-isobutyrat, E 445 Glycerinester aus Wurzelharz, E 450 Diphosphate, E 450i Dinatriumdiphosphat, E 450ii Trinatriumdiphosphat, E 450iii Tetranatriumdiphosphat, E 450iv Dikaliumdiphosphat, E 450v Tetrakaliumdiphosphat, E 450vi Dicalciumdiphosphat, E 450vii Calciumdihydrogendiphosphat, E 451 Triphosphate, E 451i Pentanatriumtriphosphat, E 451 ii Pentakaliumtriphosphat, E 452 Polyphosphate, E 452i Natriumpolyphosphat, E 452ii Kaliumpolyphosphat, E 452iii Natriumcalciumpolyphosphat, E 452iv Calciumpolyphosphat, E 460 Cellulose, E 460i mikrokristalline Cellulose, E 460ii Cellulosepulver, E 461 Methylcellulose, E 463 Hydroxypropylcellulose, E 464 Hydroxypropylmethylcellulose, E 465 Methylethylcellulose, E 466 Carboxymethylcellulose, E 469 enzymatisch hydrolysierte Carboxymethylcellulose, E 470a Natrium-, Kalium- und Calciumsalze von Fettsäuren, E 470b Magnesiumsalze von Fettsäuren, E 471 Mono- und Diglyceriden von Fettsäuren, E 472a Essigsäureester von Mono- und Diglyceriden von Fettsäuren, E 472b Milchsäureester von Mono- und Diglyceriden von Fettsäuren, E 472c Zitronensäureester von Mono- und Diglyceriden von Fettsäuren, E 472d Weinsäureester von Mono- und Diglyceriden von Fettsäuren, E 472e Mono- und Diacetylweinsäureester von Mono- und Diglyceriden von Fettsäuren, E-472f gemischte Essig- und Weinsäureester von Mono- und Diglyceriden von Fettsäuren, E 473 Zuckerester von Fettsäuren, E 474 Zuckerglyceride, E 475 Polyglycerinester von Fettsäuren, E 476 Polyglycerin-Polyricinoleat, E 477 Propylenglycolester von Fettsäuren, E 479 Thermooxidiertes Sojaöl mit Mono- und Diglyceriden von Fettsäuren, E 481 Natriumstearoyl-2-lactylat, E 482 Calciumstearoyl-2-lactylat, E 483 Stearyltartrat, E 491 Sorbitanmonostearat, E 492 Sorbitantristearat, E 493 Sorbitanmonolaurat, E 494 Sorbitanmonooleat oder E 495 Sorbitanmonopalmitat.

Unter Stabilisatoren sind Substanzen zu verstehen, die die Konsistenz oder die Zusammensetzung von Lebensmitteln erhalten. Beispielhaft seien genannt: Ascorbinsäure (E 300), Carbamit (E 927b), Eisen-II-laktat (E 585), Eisenglukonat (E 579), Glyzerinester (E 445), Lezithin (E 322), Meta-Weinsäure (E 353), Pektin (E 440), Saccharoseacetat-isobutyrat (E 444) und Zinn-II-Chlorid (E 512)

Konservierungsmittel sind Stoffe die die Haltbarkeit von Lebensmitteln verlängern, indem sie sie vor den schädlichen Auswirkungen von Mikroorganismen schützen. Beispielhaft seien genannt: E 200 Sorbinsäure, E 201 Natriumsorbat, E 202 Kaliumsorbat, E 203 Calciumsorbat, E 210 Benzoesäure, E 211 Natriumbenzoat, E 212 Kaliumbenzoat, E 213 Calciumbenzoat, E 214 Ethyl-p-hydroxybenzoat / PHB-Ester, E 215 Natrium-ethyl-p-hydroxybenzoat PHB-Ethylester Natriumsalz, E 216 Propyl-p-hydroxybenzoat / PHB-Propylester, E 217 Natriumpropyl-p-hydroxybenzoat / PHB-Propylester Natriumsalz, E 218 Methyl-p-hydroxybenzoat / PHB-Methylester, E 219 Natriummethyl-p-hydroxybenzoat / PHB- Methylester Natriumsalz, E 220 Schwefeldioxid, E 221 Natriumsulfit, E 222 Natriumhydrogensulfit / Natriumbisulfit, E 223 Natriummetabisulfit / Natriumdisulfit, E 224 Kaliummetabisulfit / Kaliumsulfit, E 226 Calciumsulfit, E 227 Calciumhydrogensulfit, E 228 Kaliumhydrogensulfit / Kaliumbisulfit, E 230 Biphenyl / Diphenyl, E 231 Orthophenylphenol, E 232 Natriumorthophenylphenol, E 233 Thiabendazol, E 234 Nisin, E 235 Natamycin, E 239 Hexamethylentetramin, E 242 Dimethyldicarbonat, E 249 Kaliumnitrit, E 250 Natriumnitrit, E 251 Natriumnitrat und E 252 Kaliumnitrat.

Bei Antibackmitteln im Sinne der vorliegenden Erfindung handelt es sich um natürlich vorkommende oder künstlich hergestellte Substanzen, die die Rieselfähigkeit eines Lebensmittels erhöhen, indem sie das Zusammenballen und Verkleben der Teilchen verhindern. Beispielhaft seien genannt: E 530 Magnesiumoxid, E 535 Natriumferrocyanid, E 536 Kaliumferrocyanid, E 541 saures Natriumaluminiumphosphat, E 551 Siliciumdioxid, E 552 Calciumsilicat, E 553ai Magnesiumsilicat, E 553aii Magnesiumtrisilicat (asbestfrei), E 553b Talkum (Asbestfrei), E 554 Natriumaluminiumsilicat und E 556 Calciumaluminiumsilicat

Unter Geschmacksverstärker in Sinne dieser Erfindung werden natürlich vorkommende oder künstlich hergestellte Substanzen verstanden, die den Geschmack von Lebensmitteln abrunden oder zu verstärken vermögen. Zu diesen werden auch die Aromen gerechnet. Beispielhaft seien genannt: E 620 Glutaminsäure, E 621 Mononatriumglutamat, E 622 Monokaliumglutamat, E 623 Calciumdiglutamat, E 624 Monoammoniumglutamat, E 625 Magnesiumdiglutamat, E 626 Guanylsäure, E 627 Dinatriumguanylat, E 628 Dikaliumguanylat, E 629 Calciumguanylat, E 630 Inosinsäure, E 631 Dinatriuminosinat, E 632 Dikaliuminosinat, E 633 Dicalciuminosinat, E 634 Calcium-5-ribonucleotid, E 635 Dinatrium-5-ribonucleotid, E 640 Glycin und E 650 Zinkacetat.

In einer Ausführungsform kann die erfindungsgemäß verwendete Zubereitung Zuschlagstoffe enthalten. Unter Zuschlagstoffen werden erfindungsgemäß Stoffe verstanden, die der Verbesserung der Produkteigenschaften, wie Staubverhalten, Fließeigenschaften, Wasseraufnahmefähigkeit und Lagerstabilität dienen. Zuschlagstoffe können auf der Basis von Zuckern z.B. Lactose oder Maltodextrin, auf der Basis von Getreide- oder Hülsenfruchtprodukten z.B. Maisspindelmehl, Weizenkleie und Sojaschrot, auf der Basis von Mineralsalzen u.a. Calcium-, Magnesium-, Natrium-, Kaliumsalze, sowie auch D-Pantothensäure oder deren Salze selbst (chemisch oder fermentativ hergestelltes D-Pantothensäuresalz) sein.

In einer weiteren Ausführungsform kann die erfindungsgemäß verwendete Zubereitung Träger enthalten. Als Träger eignen sich "inerte" Trägermaterialien, d.h. Materialien die keine negativen Wechselwirkungen mit den in der erfindungsgemäßen Zubereitung eingesetzten Komponenten zeigen. Selbstverständlich muss das Trägermaterial für die jeweilige Verwendungen als Hilfsstoff, z.B. in Nahrungsmitteln und Tierfuttermitteln, unbedenklich sein. Als Trägermaterialien eigenen sich sowohl anorganische als auch organische Träger. Als Beispiele für geeignete Trägermaterialien sind zu nennen: niedermolekulare anorganische oder organische Verbindungen sowie höhermolekulare organische

Verbindungen natürlichen oder synthetischen Ursprungs. Beispiele für geeignete niedermolekulare anorganische Träger sind Salze, wie Natriumchlorid, Calciumcarbonat, Natriumsulfat und Magnesiumsulfat Kieselgur oder Kieselsäure bzw. Kieselsäurederivate, z.B. Siliziumdioxide, Silicate oder Kieselgele. Beispiele für geeignete organische Träger sind insbesondere Zucker, z. B. Glucose, Fructose, Saccharose sowie Dextrine und Stärkeprodukte. Als Beispiele für höhermolekulare organische Träger sind zu nennen: Stärke- und Cellulosepräparate, wie insbesondere Maisstärke, Maisspindelmehl, gemahlene Reishüllen, Weizengrieskleie oder Getreidemehle, wie z. B. Weizen-, Roggen-, Gersten- und Hafermehl oder -Kleie und Gemische davon.

Die erfindungsgemäß verwendeten Zubereitungen können die weiteren Bestandteile, Träger und Zuschlagsstoffe in Mischungen enthalten.

Der Gewichtsanteil der Verbindungen T in den Zubereitungen kann in weiten Bereichen variieren und richtet sich im Allgemeinen nach praktischen Erwägungen die sich aus dem gewählten Anwendungsbereich (z.B. Nutztier-, Haustierhaltung oder Humanernährung) ergeben.

In einer Ausführungsform der vorliegenden Erfindung können die zur erfindungsgemäßen Verwendung geeigneten Zubereitungen z.B. von 5 bis 10 Gewichtsprozent (Gew.-%), bevorzugt von 10 bis 20 Gew.-%, besonders bevorzugt von 20 bis 30 Gew.-%, ganz besonders bevorzugt von 30 bis 40 Gew.-% Verbindungen T enthalten. Es können aber auch höhere Prozentgewichtsanteile gewählt werden.

Die Herstellung der erfindungsgemäß verwendeten Zubereitungen erfolgt im einfachsten Fall durch Mischen der Bestandteile. Ebenso kann die Herstellung durch Mischen von Lösungen der einzelnen Komponenten erfolgen und gegebenenfalls sich anschließender Entfernung von Lösungsmitteln.

Die Mischungen verschiedener Bestandteile können untereinander in beliebigen Gewichtsverhältnissen vorliegen.

Die einfachste Form der Mischung ist das Zusammenbringen der Bestandteile in einem Mischer. Solche Mischer sind dem Fachmann bekannt, beispielsweise von der Firma Ruberg (Vertikal-Zweiwellenmischer (Typ HM (10-50.000 l)), Ringschicht-Mischgranulator (Typ RMG), Konti Agglomerator Trockner (Typ HMTK), Vertikal-Einwellenmischer (Typ VM (10-50.000 l)), Containermischer (Typ COM (50-4000 l)). Weitere Mischer können z.B. auch von den Firmen Lödige, Drais, Engelsmann bezogen werden . Die Mischer können diskontinuierlich oder kontinuierlich betrieben werden. Im diskontinuierlichen Mischer werden im Allgemeinen alle zu mischenden Bestandteile im gewünschten Verhältnis vorgelegt und dann eine hinreichende Zeit im Bereich von Minuten bis Stunden gemischt. Die Mischzeit und die Mischbeanspruchung werden so festgelegt, dass die Bestandteile homogen verteilt in der Mischung vorliegen. Im Fall der kontinuierlichen Mischung werden die Bestandteile kontinuierlich zugegeben, gegebenenfalls nach einer Vormischung. Auch im kontinuierlichen Mischer ist die Verweilzeit und Mischbeanspruchung so zu wählen, dass die Bestandteile homogen verteilt in der Mischung vorliegen. Die Mischzeit ist im kontinuierlichen Fall häufig kürzer und die Beanspruchung höher als im Fall der diskontinuierlichen Mischung. Die Mischung wird üblicherweise bei Raumtemperatur durchgeführt, kann aber auch in Abhängigkeit der verwendeten Substanzen bei höheren oder niedrigeren Temperaturen durchgeführt werden.

In einer bevorzugten Ausführungsform liegen die Zubereitungen in fester Form vor. Je nach anwendungstechnischer Anforderung können die Zubereitungen als Pulver mit einer mittleren Partikelgröße von 10 $\mu$m bis 5000 $\mu$m, bevorzugt mit einer mittleren Partikelgröße von 20 $\mu$m bis 1000 $\mu$m.

Die erhaltene Partikelverteilung der pulverförmigen Produkte kann in einem Gerät der Firma Malvern Instruments GmbH, Mastersizer S, untersucht werden.

Mischungen von Bestandteilen sind als reine Blends möglich, das heißt, die Substanzen werden in den gewünschten Partikelgrößen und Konzentrationsverhältnissen, ggfs. unter Zugabe von weiteren Additiven, zusammengemischt, wobei Substanzen auch durch z.B. ein Coating soweit erforderlich geschützt sein können. Weiterhin sind Kern-Schale-Strukturen einsetzbar, d.h. ein Bestandteil befindet sich als Kern innen und ein weiterer Bestandteil als Schale außen - oder umgekehrt. Selbstverständlich werden auch bei diesen Strukturen weitere Umhüllungen eingesetzt, soweit dies erforderlich ist. Auch ist es denkbar, Substanzen zusammen in einer gemeinsamen Matrix aus Trägermaterialen oder Schutzcolloiden zu verkapseln. Beispiele hierfür sind dem Fachmann bekannt und z.B. in R.A. Morten: Fat-Soluable Vitamins, Pergamon Press, 1970, Seite 131 bis 145, beschrieben.

Die Herstellung der Pulver kann durch dem Fachmann geläufige Kristallisations-, Fällungs-, Trocknungs-, Granulations- oder Agglomerationsverfahren oder sonstige in den gängigen Lehrbüchern beschriebenen Verfahren zur Bildung von Feststoffen erfolgen.

Unter einem tierischen Organismus im Sinne der vorliegenden Erfindung werden die taxonomisch dem Reich der Tiere (Animalia) zugeordneten Organismen verstanden.

Dabei sind die Wirbeltiere (vertebrata) mit den Reihen der Tetrapoda (Landwirbeltiere) und Fische (Pisces) bevorzugt. Besonders bevorzugt sind die Klassen Aves (Vögel) und Mammalia (Säugetiere), wobei der modernde Mensch *(Homo sapiens)* als besonders bevorzugtes Säugetier umfasst ist.

Ganz besonders bevorzugt sind die Familien der Echten Schweine (Suidae), Rinder *(Bovinae),* Fasanenartige (Phasianidae), Entenvögel (Anatidae), Pferde (Equidae), Karpfenfische (Cyprinidae) und Forellenfische (Salmonidae).

Aus diesen Familien sind am meisten bevorzugt, die sog. Haus- und Nutztiere. Unter Haustieren im Sinne der vorliegenden Erfindung werden nicht frei lebende, an den Menschen gewöhnte Tiere verstanden werden, die von Menschen überwiegend im Wohnhaus gehalten werden. Besonders bevorzugte Haustiere sind Katzen und Hunde.

Unter Nutztieren im Sinne der vorliegenden Erfindung werden Tiere verstanden, die vom Menschen zu wirtschaftlichen Zwecken gehalten werden.

Besonders bevorzugte Nutztiere sind die Arten, Hausrind *(Bos taurus),* Haushuhn *(Gallus gallus domesticus),* Hausschwein, Hausschaf *(Ovis ammon aries)* und domestizierte Formen der Graugans, *(Anser anser).*

Der Proteinanteil eines tierischen Organismus ist zu verstehen als die Summe aller in einem Organismus vorkommenden Proteinbestandteile und umfasst sowohl lösliche (z.B. Enzyme, Hormone) als auch gebundene und fibrilläre (faserige) Strukturproteine wie Aktin, Myosin und Tropomyosin, die die Kontraktion der Muskelzellen bewirken.

Unter Steigerung des Proteinanteils eines tierischen Organismus ist die Zunahme des durch den Proteinanteil begründeten Gewichtes eines tierischen Organismus an dessen Gesamtgewicht zu verstehen.

Eine Steigerung des Proteinanteils im Sinne der vorliegenden Erfindung ist z.B. die Zunahme des durch den Proteinanteil begründeten Gewichtes eines tierischen Organismus an dessen Gesamtgewicht ausgedrückt in Prozent (im folgenden als PPGW bezeichnet (Prozentanteil des Proteinanteils am Gesamtgewicht eines Organismus)). Dabei kann die Zunahme des PPGW z.B. wie folgt kalkuliert werden:

(i) Ermittlung der Differenz des PPGW vor der Zuführung/Aufnahme und nach Beendigung der Zuführung/Aufnahme von Verbindungen T enthaltenden Zubereitungen, wobei die Zubereitungen so verabreicht werden müssen, dass dem tierischen Organismus pro Tag mindestens 0,05 g einer Verbindung T pro kg Körpergewicht über einen Zeitraum von mindestens 7 Tagen zugeführt werden;

(ii) Vergleich der PPGW's von a) tierischen Organismen denen pro Tag mindestens 0,05 g einer Verbindung T pro kg Körpergewicht über einen Zeitraum von mindestens 7 Tagen zugeführt werden und b) tierischen Organismen die einer Kontrollgruppe angehören und Zubereitungen verabreicht bekommen, bei denen lediglich die Verbindungen T durch eine Ersatzsubstanz wie z.B. ein Zuschlagstoff ersetzt wurde.

Bei diesen Messungen ist zu berücksichtigen, dass a) eine zur statistischen Absicherung der erhaltenden Ergebnisse ausreichende Anzahl an tierischen Organismen untersucht werden muß und b) alle Messungen mit der gleichen Methode durchgeführt werden. Die zur Absicherung statistischer Aussagen notwendigen Populationsgrößen und statistischen Methoden sind dem Fachmann bekannt.

Zur Bestimmung des PPGW können destruktive Meßmethoden angewendet werden. Destruktive Meßmethoden erfordern die vorherige Schlachtung des zu analysierenden Tieres.

Um dabei eine Zunahme des Proteinanteils statistisch signifikant nachweisen zu können, ist es notwendig, im Vorfeld der Zufütterung der Verbindungen T, anhand einer ausreichend großen Population von Tieren, den für diese Tiere unter den gewählten Haltungsbedingungen (Haltungsdichte, Alter, Futtermenge, Futterqualität, Geschlecht, Haltungstemperatur usw.) durchschnittlichen PPGW einer Tierart zu bestimmen. In diesem Fall kann der nach Zuführung der Verbindungen T erzielte und mit der gleichen destruktiven Methode ermittelte individuelle PPGW eines Tieres mit den vorab erzielten Mittelwerten für nicht mit Verbindungen T gefütterte Tiere verglichen werden und eine Zunahme anhand einer signifikanten Abweichung vom Mittelwert festgestellt werden.

Die erfindungsgemäße Steigerung des Proteinanteils eines Tieres kann an der Zunahme des Quotienten gebildet aus den Gewichtsprozentanteilen des Protein- und Fettanteils (Gewichtsprozentanteil Protein/ Gewichtsprozentanteil Fett, im folgenden als P/F-Quotient bezeichnet) erkannt werden.

Die Steigerung des P/F-Quotienten liegt bei erfindungsgemäßer Anwendung der Zubereitungen sowohl in der Steigerung des Proteinanteils als auch in einer koinzident ablaufenden Abnahme des prozentualen Fettanteils am Gesamtkörper-gewicht begründet.

Dabei kann die Zunahme des P/F-Quotienten z.B. wie folgt kalkuliert werden:

(iii) Ermittlung des Prozentanteil des Protein- und Fettanteils am Gesamtgewicht eines Tieres und der Bildung und Vergleich der P/F-Quotienten, die aus den Werten vor der Zuführung/Aufnahme und nach Beendigung der Zuführung/ Aufnahme von Verbindungen T enthaltenden Zubereitungen ermittelt werden, wobei die Zubereitungen wie unter (i) beschrieben zugeführt werden;

(iv) Vergleich der P/F-Quotienten von a) Testtieren und b) Kontrolltieren wie unter (ii) beschrieben.

Auch bei diesen Messungen sind die zur Absicherung statistischer Aussagen notwendigen und dem Fachmann bekannten Populationsgrößen und statistischen Methoden zu verwenden.

Ferner können auch zur Bestimmung des P/F-Quotienten destruktive Methoden angewendet werden. Um

dabei eine Zunahme des P/F-Quotienten statistisch signifikant nachweisen zu können, ist es notwendig, wie oben für die Bestimmung der PPGW beschieben, im Vorfeld der Zufütterung der Verbindungen T, die durchschnittlichen P/F-Quotienten eines Tieres zu bestimmen. In diesem Fall kann der nach Zuführung der Verbindungen T erzielte und mit der gleichen destruktiven Methode ermittelte individuelle oder durchschnittliche P/F-Quotient eines Tieres mit den vorab erzielten Mittelwerten für nicht mit Verbindungen T gefütterte Tiere verglichen werden und eine Zunahme anhand einer signifikanten Abweichung vom Mittelwert festgestellt werden.

Eine Änderung des PPGW und des P/F-Quotienten kann auch eine Änderung der sogenannten lean body mass zur Folge haben.

Eine erfolgreiche Anwendung der erfindungsgemäßen Zubereitungen kann demnach aus der Steigerung des durch den Proteinanteil begründeten Gewichtes eines tierischen Organismus an dessen Gesamtgewicht, als auch an einer Zunahme des Magerfleisch- bzw. Muskelfleischanteils und somit an einer Steigerung der lean body mass erkannt werden.

Methoden zur Ermittlung der Schlachtkörperzusammensetzung und der lean body mass sind dem Fachmann hinlänglich bekannt. Dabei kann man zwischen destruktiven (am geschlachteten Tier vorgenommen Messungen) und nicht destruktiven Methoden (am lebenden Tier vorgenommen Messungen) unterscheiden.

Destruktive Methoden:

Unter Verwendung eines optischen Verfahrens kann an einer Probe der Fleischanteil (Optical Grading Probe oder Fibre Optic) am unzerlegten Schlachtkörper geschätzt werden. Für diese Messungen können Fat-O-Meat'er (FOM) Geräte eingesetzt werden. Diese Geräte ermitteln mit einem optischen Verfahren die Speck- und Muskeldicke und schätzen den Muskelfleischanteil des Schlachtkörpers.

Es können aber auch speziell entwickelte elektromagnetische Scanner zur Schätzung der Schlachtkörperzusammensetzung verwendet werden. Für dieses Verfahren wird häufig auch die Abkürzung TOBEC (Total Body Electrical Conductivity) verwendet. Die Schlachtkörper werden dabei durch ein Magnetfeld geführt. Da Muskel- und Fettgewebe unterschiedliche Leitfähigkeiten besitzen, wird die Energie im Magnetfeld je nach Gewebeanteilen unterschiedlich stark absorbiert.

Nicht destruktive Methoden:

Für die Beurteilung der Fleischigkeit am lebenden Tier stehen verschiedene Methoden zur Verfügung. Am häufigsten wird das Ultraschall- oder Echolot-Verfahren eingesetzt.

Ein weiteres Verfahren den Fleischanteil sowohl im lebenden Tier wie auch im Schlachtkörper zu schätzen, ist die Bioelektrische Impedanz.

Das Verfahren misst die Leitfähigkeit des Tierkörpers für einen Wechselstrom und leitet daraus die Körperzusammensetzung ab.

Weitere Technologien zur Schätzung der Körperzusammensetzung sind die Röntgen-Computer-Tomographie (CT) und die Magnet-Resonanz-Spektroskopie (nuclear magnetic resonance, NMR). Diese Messtechniken lassen im Vergleich zu Ultraschallgeräten weit höhere Messgenauigkeiten zu. So ist es möglich, bereits geringe Veränderungen der Körperzusammensetzung des einzelnen Tieres zuverlässig zu messen.

Die Körperfettanalyse beim Menschen kann ebenfalls unter Verwendung der Bioelektrische Impedanz mittels sogenannter Körperfettanalysewaagen durchgeführt werden.

Zur Steigerung des Proteinanteils eines tierischen Organismus kann die Anwendung der erfindungsgemäßen Zubereitungen so durchgeführt werden, dass die Verabreichungsdosen der Verbindungen T pro Tag (24 Std.) von 0,01 g bis 20 g, bevorzugt von 0,02 g bis 15 g, besonders bevorzugt von 0,03 g bis 10 g, ganz besonders bevorzugt von 0,04 g bis 8 g und am meisten bevorzugt von 0,05 g bis 6 g pro kg des Gewichtes des tierischen Organismus ausmachen. Es können aber auch höhere Dosen der erfindungsgemäßen Zubereitungen verabreicht werden.

Dabei kann die zur erfindungsgemäßen Verwendung notwendige Menge der Zubereitung auch in mehreren kleineren Portionen verteilt über einen Tag erfolgen.

Zur erfindungsgemäßen Steigerung des Proteinanteils eines tierischen Organismus muß eine Verbindung T in der oben beschriebenen Menge mindestens über einen Zeitraum von 7 Tagen, bevorzugt 14 Tagen, besonders bevorzugt 21 Tagen, ganz besonders bevorzugt 28 Tagen verabreicht werden. Es können aber auch geeignete Dosen der erfindungsgemäßen Verbindungen T über einen längeren Zeitraum verabreicht werden, z.B. über die gesamte Lebensdauer des Tieres.

In einer weiteren bevorzugten Ausführungsform der vorliegenden Erfindung werden die Verbindungen T oder diese enthaltende Zubereitungen, Tierfutter oder menschlichen Nahrungsmitteln zugesetzt.

Besonders bevorzugt ist dabei die erfindungsgemäße Verwendung der Zubereitungen in Tierfutter für Nutz- und Haustiere wie z.B. Schweine, Geflügel, Kälber bzw. Katzen und Hunde.

Die Zubereitungen können dabei in Form von Flüssigkeiten, Pulvern, Vormischungen, Tabletten, Kapseln oder Suspensionen dem Futter zugegeben werden.

In einer Ausführungsform werden die Zubereitungen unabhängig von der Nahrung als Flüssigkeit, Pulver, Tablette, Kapsel oder Suspension den tierischen Organismen zusätzlich, aber getrennt von der normalen Nahrung verabreicht.

Ein weiterer Gegenstand der Erfindung ist die Verwendung der erfindungsgemäßen Zubereitungen in der Humanernährung als therapeutisches Mittel.

Unter therapeutischen Mitteln werden im Sinne dieser Erfindung Mittel verstanden, die sowohl zur Körpergewichtsreduktion aber auch zum Muskelaufbau z.B. im Breiten- und Leistungssport, im speziellen beim Body Building eingesetzt werden. Dabei ist explizit die erfindungsgemäße Verwendung der Zubereitungen in dietätischen Lebensmittel oder functional food mit eingeschlossen.

Ferner bewirken therapeutische Mittel eine Verbesserung der lean body mass beim Menschen. In einer Ausführungsform werden unter therapeutischen Mitteln solche Mittel verstanden, die sowohl zur Vorbeugung als auch zur therapeutischen Behandlung von ernährungsbedingter, genetisch bedingter oder durch Stoffwechselstörungen hervorgerufener Adipositas eingesetzt werden. Bei der therapeutischen Behandlung von Adipositas können die Zubereitungen der vorliegenden Erfindung in einer dem Fachmann allgemein bekannten und geeigneten Weise formuliert werden und zur Herstellung pharmazeutischer Darreichungsformen unter Anwendung von konventionellen Techniken verwendet werden. Deratige Techniken sind z.B. in "Remington's Pharmaceutical Science Handbook, Mack Publishing Co., New York, USA, 17 Auflage 1985 beschrieben. Bei derartigen pharmazeutischen Darreichungsformen oder Nahrungsmittelzusatzstoffen kann es sich um Flüssigkeiten, Pulver, Vormischungen, Tabletten, Kapseln oder Suspensionen handeln.

So konnte gezeigt werden, dass Verbindungen T enthaltende Zubereitungen einen nachhaltigen physiologischen Effekt auf den tierischen Stoffwechsel dergestalt haben, dass der Proteinanteil zunimmt (Steigerung des PPGW). Ferner konnte beobachtet werden, dass infolge der Verabreichung der erfindungsgemäßen Zubereitungen, die Zunahme des Gesamtkörperproteinanteils mit einer Verminderung des Gesamtkörperfettanteils einhergeht (Steigerung des P/F-Quotienten).

Es wurde gefunden, dass die erfindungsgemäßen Zubereitungen sich insbesondere für die Verwendung in der Tierernährung eignen.

Beispiel: Untersuchungsmethoden:

1. Bestimmung von Gesamtkörper-Rohprotein

1) Methode zur Bestimmung des Gehaltes an Rohprotein in Mäusen aus dem Stickstoffgehalt, modifiziert nach Kjeldahl (Kjeldahl, J.: Z. anal. Chem. 22, 366-382. 1883).

2) Prinzip

Die Versuchstiere wurden geschlachtet und nach Entfernung des Margendarmtraktes wurde das Gewicht der Tiere durch wiegen ermittelt. Anschließend wurden die Versuchstiere in flüssigem Stickstoff schockgefroren und homogenisiert. Die so erhaltenen Proben wurden auf nassem Wege aufgeschlossen. Der saure Aufschluss wurde mit Natronlauge alkalisiert. Das freigesetzte Ammoniak wurde durch Destillation abgetrennt und in einer bestimmten Menge Schwefelsäure aufgefangen, deren Überschuss mit Natronlauge titriert wurde.

3) Reagentien

3.1) Kaliumsulfat, p. a,

3.2) Katalysator: Kupfer(II)-oxid, CuO, p. a., oder kristallisiertes Kupfersulfat, $CuSO_4$* $5H_2O$, p. a., oder Quecksilber, oder Quecksilberoxid, HgO, p, a.

3.3) Schwefelsäure;, p. a., D 1,84

3.4) Bimsstein, gekörnt, mit Salzsäure gewaschen und geglüht

3.5) Zink, p. a., gekörnt

3.6) Schwefelsäure, 0,1 N; 3.7) Schwefelsäure, 0,5 N

3.8) Methylrot-Indikator: 300 mg Methylrot werden in 100 ml Ethanol, 94 bis 96%ig (V/V) gelöst.

3.9) Natronlauge, p. a., 40%ig (G/V)

3.10) Natriumsulfidlösung, p. a., gesättigt

3.11) Natriumthiosulfatlösung, 8 %ig (GN), $Na_2S_2O_3$* $5H_2O$, p. a.

3.12) Natronlauge, 0,1 N; 3.13) Natronlauge, 0,25 N

4) Geräte
Aufschlussapparatur und Destillationsapparatur nach Kjeldahl (s.o.);
5) Aufschluss
1 g der Probe wurde auf 1 mg genau eingewogen und in den Kolben der Aufschluss-Apparatur gebracht. 10 g Kaliumsulfat (3.11), eine geeignete Menge des Katalysators (3.2) (0,3 bis 0,4 g Kupferoxid oder 0,9 bis 1,2 g Kupfersulfat oder ein Tropfen Quecksilber oder 0,6 bis 0,7 g Quecksilberoxid), 25 ml Schwefelsäure (3.3) und ein paar Körner Bimsstein (3,4) wurden hinzugefügt, und der Kolbeninhalt wurde gemischt. Der Kolben wurde zunächst mäßig, dann - unter Schütteln von Zeit zu Zeit - bis zur Verkohlung der Substanz und zum Verschwinden des Schaums und schließlich intensiv bis zum gleichmäßigen Sieden der Flüssigkeit erhitzt. Eine Überhitzung der Kolbenwände und ein Festsetzen organischer Teile an den Kolbenwänden wurde vermieden. Sobald die Lösung klar und farblos war, (bei Verwendung eines kupferhaltigen Katalysators hellgrün) wurde diese noch 1 Std. weiter im Sieden gehalten; danach lies man abkühlen.
6) Destillation
Unter fortlaufendem Schwenken wurden vorsichtig 250 bis 356 ml Wasser zugegeben, wobei sich die Sulfate vollständig lösen sollten. Man lies abkühlen; sodann setzte man einige Körnchen Zink (3.5) zu. In den Auffangkolben der Destillationsapparatur wurden je nachdem zu erwartenden Stickstoffgehalt zwischen 10 ml und 25 ml Schwefelsäure (3.6) oder (3.7) gegeben und einige Tropfen Methylrot-Indikator (3.8) hinzugefügt. Der Kolben wurde mit dem Kühler der Destillationsapparatur verbunden und dessen äußerstes Ende mindestens 1 cm tief in die Flüssigkeit des Auffangkolbens gesenkt. Durch den Hahntrichter wurden 100 ml Natronlauge (3.9) langsam in den Destillierkolben eingefüllt. Bei Verwendung eines Quecksilber-Katalysators wurden in den Destillierkolben außerdem 10 ml Natriumsulfidlösung (3.1 0) oder 25 ml Natriumthiosulfatlösung (3.11) hinzugefügt. Der Kolben wird so erhitzt, dass in 30 Min. etwa 150 ml der Flüssigkeit abdestilliert wurden. Nach Ablauf dieser Zeit wurde die neutrale Reaktion des übergehenden Destillats mit Lackmuspapier kontrolliert. War die Reaktion alkalisch, so wurde die Destillation fortgesetzt. Sie wurde beendet, wenn das übergehende Destillat sich auf dem Lackmuspapier als neutral erwies. Während des Destillationsvorgangs wurde der Inhalt des Auffangkolbens von Zeit zu Zeit geschwenkt und seine Färbung beobachtet. Wenn sie ins Gelbliche umschlug, wurde sofort eine genau abgemessene Menge Schwefelsäure (3.6) oder (3.7) zugesetzt.
7) Titration
In dem Auffangkolben wurde der Überschuss an Schwefelsäure mittels Natronlauge (3.12) oder (3.13), je nach Normalität der verwendeten Schwefelsäure, bis zum Umschlag der Färbung zu klarem Gelb titriert.
8) Kontrolle der Methode
Zur Feststellung, ob die Reagentien stickstofffrei war, wurde ein Blindversuch (Destillation und Titration) ohne die zu analysierende Probe vorgenommen. Zur Kontrolle der Genauigkeit der Methode wurde die Analyse (Aufschluss, Destillation und Titration) mit 1,5 bis 2,0 g Azetanilid, p. a., (Schmelzpunkt: 114° C, %N: 10,36) bei Anwesenheit von 1 g Stickstofffreier Saccharose vorgenommen. 1 g Acetanilid verbrauchte 14,80 ml Schwefelsäure 0,5 N.
9) Berechnung der Ergebnisse
Die Menge der verbrauchten Schwefelsäure wurde ermittelt. 1 ml Schwefelsäure 0,1 N entspricht 1,4 mg Stickstoff. Die Stickstoffmenge wurde mit dem Faktor 6,25 multipliziert. Das Ergebnis wurde in Prozenten der Probe ausgedrückt.
10) Wiederholbarkeit
Der Unterschied zwischen zwei Parallelbestimmungen durfte bei derselben Probe bei Gehalten von weniger als

| | |
|---|---|
| 20 % Rohprotein | 0,2 % absolut, |
| 20 % bis 40 % Rohprotein | 1,0 % relativ, |
| mehr als 40 % Rohprotein | 0,4 % absolut |

nicht überschreiten.Weitere geeignete Methoden und Hinweise zur Analyse zur Rohproteinbestimmung kann folgenden Schriften entnommen werden: "Die chemische Untersuchung von Futtermitteln", Methodenbuch III; Naumann, K., Neumann-Neudamm, J.; 1982; "Untersuchungen über die Stickstoffbestimmung nach Kjehldahl". Rufeger, H., Z. Tierphysiol., Tierernähr., Futtermittelkunde 22, 49, 1966/67; s. 2. ibid. 189-199; "Zur Rationalisierung der Rohproteinbestimmung". Dickhaut G., Mitteilungs-bl. d. GDCh - Fachgr. Lebensmittelchemie u. gerichtl. Chemie 24, 189-191, 1970)
2. Bestimmung des Gesamtkörper- Rohfett

1) Die Methode dient zur Bestimmung des Gehaltes an Rohfett in Mäusen. Da in Vorfeld der Extraktion Knochen

und andere feste Körperbestandteile nicht entfernt wurden, wurde zur Bestimmung des Gesamtkörper-Rohfettes der Versuchstiere die unter 2b) beschriebene Variante verwendet.

2) Prinzip

2a) Die - wie im Beispiel 1 beschrieben - gewonnenen Proben wurden mit Diethylether (3.2) extrahiert. Das Lösungsmittel wurde abdestilliert und der Etherextrakt getrocknet und gewogen.

2b) Bei Proben, deren Fett ohne vorherige Hydrolyse nicht vollständig mit Diethylether extrahiert werden konnte, wurde die Probe mit Salzsäure in der Siedehitze hydrolysiert. Die Lösung wurde abgekühlt und filtriert. Der gewaschene und getrocknete Rückstand wurde anschließend wie beschrieben mit Diethylether extrahiert.

3) Reagentien

3.1) Natriumsulfat, p, a., wasserfrei

3.2) Diethylether, wasserfrei D 0,720, Kp: 34,5° C, praktisch frei von Peroxiden

3.3) Salzsäure, ca. 3 N

3.4) Filtrationshilfsmittel, z. B. Kieselgur, Hyflo-supercel

3.5) Tetrachlorkohlenstoff, p. a.

4) Geräte

4.1) Extraktionsapparatur nach Soxhlet oder gleichwertiges Gerät

4.2) Heizapparat mit Temperaturregulierung, explosionsgeschützt

4.3) Vakuumtrockenschrank (weniger als 0,133 Bar)

5) Verfahren 2a)
5 g der Probe wurden auf 1 mg genau eingewogen und mit 2 bis 3 g - nötigenfalls mehr- Natriumsulfat (3.1) gemischt. Das Gemisch wurde in eine fettfreie Extraktionshülse gefüllt und in einer Extraktionsapparatur (4.1) sechs Std. mit Diethylether (3,2) extrahiert. Bei Anwendung einer Extraktionsapparatur nach Soxhlet ist die Heizung so einzustellen, dass das Lösungsmittel mindestens 15mal in der Stunde abläuft. Der Extrakt wurde in einem trockenen, mit einigen Stückchen Bimsstein versehenen, tarierten Kolben aufgefangen. Nach der Extraktion wurde der Diethylether abdestilliert, der Rückstand mit Kolben anschließend eineinhalb Std. im Vakuumtrockenschrank (4.3) bei 75°C, weniger als 0,133 Bar, getrocknet und nach dem Abkühlen im Exsiccator gewogen. Durch eine zweite Trocknung von 30 Min. Dauer vergewissert man sich, ob das Gewicht des Fettes konstant geblieben ist. Der Gewichtsverlust muss unter 1 mg bleiben.
Verfahren 2b)
2,5 g der Probe wurden auf 1 mg genau eingewogen und in ein 400-ml-Becherglas oder einen 300-ml-Erlenmeyerbolben gebracht. Anschließend wurden 100 ml Salzsäure (3.3) und einige Stückchen Bimsstein hinzugefügt. Das Becherglas wurde mit einem Uhrglas bedeckt, bzw. der Erlenmeyerkolben wurde mit einem Rückflusskühler versehen. Das Gemisch wurde auf kleiner Flamme oder auf einer Heizplatte zum Sieden erhitzt und eine Std. im leichten Sieden gehalten. Ein Festsetzen der Substanz an den Gefäßwänden wurde durch gelegentliches Umschwenken verhindert. Dann wird abgekühlt und so viel Filtrationshilfsmittel (3.4) zugesetzt, dass beim Filtrieren kein Fettverlust eintrat. Filtriert wurde unter Verwendung eines feuchten, fettfreien doppelten Papierfaltenfilters. Der Rückstand wurde bis zum Verschwinden der sauren Reaktion mit klaren Wasser gewaschen. Danach wurde geprüft, ob das Filtrat Fett enthielt. Vorhandensein von Fett im Filtrat zeigt, dass vor der Hydrolyse eine Extraktion der Probe mir Diethylether nach dem Verfahren 2a vorzunehmen ist. Der doppelte Filter mit dem Rückstand wurde zusammengeklappt auf ein Uhrglas gelegt und eineinhalb Std. bei 95 bis 98°C im Trockenschrank getrocknet. Anschließend wurden Filter samt Rückstand in eine Extraktionshülse gebracht und mit Diethylether extrahiert. Dann wurde wie in 2a beschrieben weitergearbeitet.

6) Berechnung der Ergebnisse

Das Ergebnis wurde in Prozenten der Probe ausgedrückt.

7) Wiederholbarkeit

Der Unterschied zwischen zwei Parallelbestimmungen durfte bei derselben Probe 0,3 % Rohfett nicht Überschreiten.

Bei Erzeugnissen mit hohem Fettgehalt, die schwer zu zerkleinern sind oder sich nicht zur Entnahme einer reduzierten homogenen Probe eignen, wurde folgendermaßen verfahren: 20 g der Probe wurden auf 1 mg genau abgewogen und mit mindestens 10 g Natriumsulfat (3.1) gemischt. Dann wurde mit Diethylether (3.2) nach Verfahren 2a extrahiert. Der dabei aufgefangene Extrakt wurde mit Tetrachlorkohlenstoff (3.5) auf 500 ml aufgefüllt und gemischt. Von der Lösung wurden 50 ml entnommen und in einen kleinen, trockenen, mit Bimssteinkörnern versehenen und tarierten Kolben gebracht. Das Lösungsmittel wurde abdestilliert; dann wurde nach Verfahren 2a getrocknet und weiter verfahren. Der Extraktionsrückstand in der Hülse wurde vom Lösungsmittel befreit und auf 1 mm zerkleinert. Das Material wurde in die Extraktionshülse zurückgebracht - es durfte kein Natriumsulfat hinzugefügt werden - und mit Diethylether extrahiert; dann wurde nach Verfahren 2a fortgefahren.

Das Endergebnis wurde unter Berücksichtigung des aliquoten Teiles bei der 1. Extraktion ermittelt und in Prozenten der Probe wie folgt ausgedrückt:

$$(10\,a + b) * 5$$

a = Etherextrakt in g nach der 1. Extraktion im aliquoten Teil
b = Etherextrakt in g nach der 2. Extraktion

3. Zusammensetzung des Standardfutters: Maus/Ratte (Futter kann bezogen werden von Provimi Kliba AG, CH-4303 Kaiseraugst)

| Inhaltsstoffe | Aminosäuren | Vitamine (Zugabe/Kg Futter) | Mengenelemente | Spurenelemente pro kg |
|---|---|---|---|---|
| Getreideprodukte und Energieträger: Gerste, Weizen, Weizenkleie, Weizenstärke, Mais, Sojaöl; Pflanzliche Proteinträger: Sojaextraktionsschrot, Bierhefe, Tierische Proteinträger, Geflügelfleischmehl, Molkenpulver; Mineralstoffträger für Mengenelemente: Kohlensaurer Kalk, Di-Calciumphosphat, Salz; Aminosäuren: L-Lysin, DL-Methionin Vitamin- und Spurenelementvormischungen | Arginin 1.10 %,<br>Lysin 1.00 %, Methionin 0.39%, Methionin + Cystin 0.75%, Tryptophan 0.20%. Threonin 0.65% | Vitamin A 14000 IE<br>Vitamin D3 800 IE<br>Vitamin E 80mg<br>Vitamin K3 4mg<br>Vitamin B1 20mg<br>Riboflavin 12mg<br>Nicotinsäure 36mg<br>Pantothensäure 30mg<br>Folsäure 2 mg<br>Vitamin B6 9 mg<br>Vitamin B12 0.05mg<br>Biotin 0.2 mg | Calcium 1.05%,<br>Phosphor 0.80%, verdaulicher Phosphor 0.38%, Natrium 0.20%, Kalium 0.78%, Magnesium 0.20%, Chlor 0.36% | Kupfer 14 mg<br>Zink 60 mg.<br>Eisen 250 mg<br>Jod 1 mg/kg<br>Mangan 60 mg<br>Selen 0.3 mg |

Beispiel 1

Steigerung des PPGW (Prozentanteils des Proteinanteils am Gesamtgewicht) und Zunahme des P/F-Quotienten bei Mäusen.

In einem Fütterungsversuch wurden 48 Mäuse (Typ CD-1 M (ICR) BR) in drei verschiedenen Gruppen (Gruppe 1, 2 und 3) a 16 Tiere aufgeteilt. Die Tiere wurden jeweils paarweise in Käfigen gehalten. Jedes Paar wurde gemeinsam analysiert, wodurch sich pro Gruppe 8 Replikate bestehend aus 2 Individuen ergaben. Zu Beginn des Versuches waren die Tiere 5 Wochen alt. Die Haltung erfolgte paarweise in Käfigen mit freiem Zugang zu Wasser und Futter. Als Versuchsfutter fand ein Standardfutter (siehe Beispiel 3) Verwendung.

Das Standardfutter wurde mit 3% Palmfett und 1 % Zellulose ergänzt. Dabei wurde an die Gruppe 1 (Kontrollgruppe) keine Tartronsäure verfüttert. Bei den Gruppen 2 und 3 wurden 0,25% bzw. 0,75% des Zelluloseanteils des Futters durch Tartronsäure ersetzt.

Die Zugabe von Tartronsäure erfolgte über einen Zeitraum von 21 Tagen. Nach dieser Zeit wurden die Tiere geschlachtet und der Gesamtkörperfett- bzw. Gesamtkörperproteinanteil wie oben beschrieben bestimmt.

| Behandlung/ Gruppe | Tartronsäure [Gew. Anteil des Futters] | Replikate | Anzahl der Tiere pro Replikat | mittlere Futteraufnahme [g/Tag] | mittlere Gesamtgewichtszunahme der Tiere [g/Tag] |
|---|---|---|---|---|---|
| 1 | 0 | 8 | 2 | 253,8 | 25, 7 |
| 2 | 0,25 | 8 | 2 | 250,4 | 25, 2 |
| 3 | 0,75 | 8 | 2 | 246,7 | 24,2 |

| Behandlung | Körperprotein [Gew. % Körpergewicht] | Körperfett [Gew. % Körpergewicht] | P/F-Quotient |
|---|---|---|---|
| 1 | 62,66 | 23,84 | 2,68 |
| 2 | 65,93 | 20,25 | 3,53 |
| 3 | 66,04 | 20,65 | 3,28 |

Hinsichtlich Futteraufnahme und Zuwachs ergaben sich keine signifikanten Unterschiede. Überrachenderweise war eine Zunahme des PPGW bei den mit Tartronsäure gefütterten Tieren zu beobachten, was auch an der Steigerung des P/F-Quotienten erkenntlich ist.

## Patentansprüche

1. Verwendung von Tartronsäure, deren Derivate oder deren Mischungen (Verbindungen T) zur Herstellung einer Zubereitung geeignet zur Steigerung des Proteinanteils eines tierischen Organismus.

2. Verwendung nach Anspruch 1, **dadurch gekennzeichnet, dass** die Zubereitung so verabreicht wird, dass dem tierischen Organismus pro Tag zwischen 0,05 und 6 g einer Verbindung T pro kg Körpergewicht zugeführt werden.

3. Verwendung nach Anspruch 2, **dadurch gekennzeichnet, dass** die Verabreichung der Zubereitung über einen Zeitraum von mindestens 7 Tagen erfolgt.

4. Verwendung nach den Ansprüchen 1 bis 3, **dadurch gekennzeichnet, dass** die Zubereitung neben einer Verbindung T weitere organische Säuren, Carotinoide, Spurenelemente, Antioxidantien, Vitamine, Enzyme, Aminosäuren, Mineralstoffe, Emulgatoren, Stabilisatoren, Konservierungsmittel, Antibackmittel, Geschmacksverstärker, Trägermaterialien und/oder Zuschlagstoffe oder Mischungen der genannten Substanzen enthält.

5. Verwendung nach Anspruch 4 **dadurch gekennzeichnet, dass** die Zubereitung Tierfutter oder menschlichen Nahrungsmitteln zugesetzt wird.

6. Verwendung nach Anspruch 5, **dadurch gekennzeichnet, dass** die Zubereitung in der Nutztier- oder Haustierhaltung eingesetzt wird.

7. Verwendung nach Anspruch 6, **dadurch gekennzeichnet, dass** es sich bei den Nutz- und Haustieren um Schweine, Geflügel, Kälber, Katzen oder Hunde handelt.

8. Verwendung nach Anspruch 5, **dadurch gekennzeichnet, dass** die Zubereitung in der Humanernährung als therapeutisches Mittel eingesetzt wird.

## Claims

1. The use of tartronic acid, its derivatives or its mixtures (compounds T) for producing a preparation suitable for increasing the protein proportion of an animal organism.

2. The use according to claim 1, wherein the preparation is administered in such a manner that between 0.05 and 6 g of a compound T is fed per day to the animal organism per kg of body weight.

3. The use according to claim 2, wherein the preparation is administered over a period of at least 7 days.

4. The use according to claims 1 to 3, wherein the preparation, in addition to a compound T, comprises further organic acids, carotenoids, trace elements, antioxidants, vitamins, enzymes, amino acids, minerals, emulsifiers, stabilizers, preservatives, anticaking agents, flavor enhancers, carriers and/or aids or mixtures of said substances.

5. The use according to claim 4, wherein the preparation is added to animal feed or human foods.

6. The use according to claim 5, wherein the preparation is used in farm animal or domestic animal husbandry.

7. The use according to claim 6, wherein the farm animals and domestic animals are pigs, poultry, calves, cats or dogs.

8. The use according to claim 5, wherein the preparation is used in human nutrition as therapeutic agent.

## Revendications

1. Utilisation d’acide tartronique, de ses dérivés ou de ses mélanges (composés T) pour la préparation d’une composition appropriée pour augmenter la proportion de protéines d’un organisme animal.

2. Utilisation selon la revendication 1, **caractérisée en ce que** la composition est administrée de manière telle que l’organisme animal reçoit par jour entre 0,05 et 6 g d’un composé T par kg de poids corporel.

3. Utilisation selon la revendication 2, **caractérisés en ce que** l’administration de la composition est réalisée sur un laps de temps d’au moins 7 jours.

4. Utilisation selon les revendications 1 à 3, **caractérisée en ce que** la composition contient, outre un composé T, d’autres acides organiques, des caroténoïdes, des oligo-éléments, des antioxydants, des vitamines, des enzymes, des acides aminés, des substances minérales, des émulsifiants, des stabilisateurs, des conservateurs, des antiagglomérants, des exhausteurs de goût, des matériaux support et/ou des additifs ou des mélanges des substances mentionnées.

5. Utilisation selon la revendication 4, **caractérisée en ce que** la composition est additionnée à des fourrages pour animaux ou des aliments pour l’homme.

6. Utilisation selon la revendication 5, **caractérisée en ce que** la composition est utilisée dans la garde d’animaux de rente ou d’animaux domestiques.

7. Utilisation selon la revendication 6, **caractérisée en ce qu’**il s’agit, pour les animaux de rente et domestiques, de porcs, de volailles, de veaux, de chats ou de chiens.

**8.** Utilisation selon la revendication 5, **caractérisée en ce que** la composition est utilisée dans l'alimentation humaine comme agent thérapeutique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2806804
- US 3988470 A
- WO 9410127 A
- WO 0048596 A


### In der Beschreibung aufgeführte Nicht-Patentliteratur


- **Wesson LG.** *Endocrinology,* Oktober 1950, vol. 47 (4), 302-4
- **Archeson et al.** *Am J Clin Nutr.,* August 1988, vol. 48 (2), 240-7
- **Chang et al.** *Arch Biochem Biophys.,* 01. August 1992, vol. 296 (2), 468-73
- **Savitha.** *World Journal of Microbiology & Biotechnology,* 2000, vol. 16, 513-518
- **Caselli et al.** *J Bone Mineral Res.,* Juli 1997, vol. 12 (6), 972-81
- **B. Bak.** *Justus Liebigs Annalen der Chemie,* 1939, vol. 537, 286
- **P.Chang ; S.L. Wu.** *Chem. Abstr.,* 1958, vol. 52, 14567
- *Tetrahedron letters,* 1998, vol. 39 (18
- **R.A. Morten.** Fat-Soluable Vitamins. Pergamon Press, 1970, 131-145
- Remington's Pharmaceutical Science Handbook. Mack Publishing Co, 1985
- **Kjeldahl, J.** *Z. anal. Chem.,* vol. 22, 366-382
- **Naumann, K. ; Neumann-Neudamm, J.** Methodenbuch III. *Die chemische Untersuchung von Futtermitteln,* 1982
- **Rufeger, H. ; Z. Tierphysiol. ; Tierernähr. ; Futtermittelkunde.** *Untersuchungen über die Stickstoffbestimmung nach Kjehldahl,* 1966, vol. 22, 49
- *UNTERSUCHUNGEN UBER DIE STICKSTOFF-BESTIMMUNG NACH KJEHLDAHL,* 189-199
- **Dickhaut G.** Zur Rationalisierung der Rohproteinbestimmung. *Mitteilungs-bl. d. GDCh - Fachgr. Lebensmittelchemie u. gerichtl. Chemie,* 1970, vol. 24, 189-191